# EUROPÄISCHE PATENTSCHRIFT

(11) **EP 3 210 549 B1**
(45) Veröffentlichungstag und Bekanntmachung des Hinweises auf die Patenterteilung: **15.01.2020**
(21) Anmeldenummer: 17164087.3
(22) Anmeldetag: 27.02.2014
(51) Int. Cl.: A61B 17/16, A61B 17/88, A61B 90/00

(54) **CHIRURGISCHES INSTRUMENT**
SURGICAL INSTRUMENT
INSTRUMENT CHIRURGICAL

(30) Priorität: 06.06.2013 DE 102013105841
(43) Veröffentlichungstag der Anmeldung: 30.08.2017
(62) Teilanmeldung aus: 14157014.3
(73) Patentinhaber: Aesculap AG, 78532 Tuttlingen (DE)
(72) Erfinder: Doser, Andreas, 79098 Freiburg (DE)

(56) Entgegenhaltungen:
- WO-A1-2013/021310
- DE-A1-102011 111 671

## Beschreibung

Die Erfindung betrifft ein chirurgisches Instrument, umfassend ein Gehäuse und eine darin angeordnete Antriebseinheit, wobei distal am Gehäuse ein chirurgisches Werkzeug zum Einwirken auf ein zu bearbeitendes Körpergewebe, insbesondere Knochen, festlegbar oder festgelegt ist, welches Werkzeug von der Antriebseinheit drehend oder oszillierend antreibbar ist und wobei bei angetriebenen Werkzeug ein Vortrieb des Instrumentes relativ zum Körpergewebe erzielbar ist.

Ein derartiges Instrument ist beispielsweise eine chirurgische Bohrvorrichtung mit einem Werkzeug in Gestalt eines chirurgischen Bohrers. Das Körpergewebe, im Fall der Bohrvorrichtung insbesondere Knochen, kann unter drehendem Antrieb des Bohrers bearbeitet werden. Infolge des Eindringens des Werkzeugs in den Knochen wird die Bohrvorrichtung relativ zum Knochen vorgetrieben. Der Vortrieb beeinflusst die Tiefe des Bohrloches, die vom Operateur insbesondere dahingehend überwacht werden muss, ob ein Implantat zuverlässig darin implantiert werden kann.

Als "Vortrieb" wird vorliegend insbesondere die Änderung des Abstandes des Instrumentes und des Körpergewebes voneinander aufgrund von dessen Bearbeitung verstanden.

Die Kontrolle der Bohrlochtiefe erweist sich mitunter als schwierig, weil sich Weichgewebe über den bearbeiteten Knochen legen und damit die Sicht auf diesen beeinträchtigen oder den Zugriff des Operateurs auf den Knochen erschweren kann. Auch Blut, das im Operationsfeld austritt, kann die Sicht erschweren. Weiter erweist es sich als nachteilig, dass der Operateur zur Kontrolle der Bohrlochtiefe den Bearbeitungsvorgang, möglicherweise sogar mehrfach, unterbrechen muss.

Weitere Beispiele für ein gattungsgemäßes chirurgisches Instrument sind eine chirurgische Fräsvorrichtung mit einem Werkzeug in Gestalt eines chirurgischen Fräskopfes oder eine chirurgische Schraubvorrichtung mit einem Werkzeug in Gestalt eines Schraubwerkzeugs (etwa eines Schraub-Bits) zum Zusammenwirken mit einer chirurgischen Schraube.

Ein gattungsgemäßes chirurgisches Instrument ist in der DE 10 2011 111 671 A1 beschrieben.

Aufgabe der vorliegenden Erfindung ist es, ein chirurgisches Instrument der eingangs genannten Art mit besserer Handhabbarkeit bereitzustellen.

Diese Aufgabe wird erfindungsgemäß durch ein chirurgisches Instrument mit den Merkmalen von Anspruch 1 gelöst.

Die Vortriebermittlungseinrichtung ermöglicht es, den Vortrieb und damit eine Abstandsänderung des Instrumentes relativ zum Körpergewebe im Betrieb des Instrumentes zu ermitteln. Zu diesem Zweck umfasst die Vortriebsermittlungseinrichtung die Sendeeinheit, die Empfangseinheit und die Auswerteeinheit, wobei anhand mindestens eines ausgesandten und empfangenen Signals von der Auswerteeinheit der Vortrieb ermittelt werden kann. Dem Operateur bleibt es erspart, die Bearbeitung des Körpergewebes zu unterbrechen. Dadurch erweist sich das chirurgische Instrument zum Einen als benutzer- und handhabungsfreundlicher, weil der Operateur während der Bearbeitung feststellen kann, ob der Vortrieb des Instrumentes ausreichend für den jeweils zu erzielenden Einsatz ist. Beispielsweise kann anhand des Vortriebs auf die Tiefe eines Bohrloches im Falle einer chirurgischen Bohrvorrichtung, die Stärke abgefrästen Materials im Falle einer chirurgischen Fräsvorrichtung oder die Einschraubtiefe im Falle einer chirurgischen Schraubvorrichtung ermittelt werden. Zum Anderen erweist sich das Instrument als patientenfreundlicher. Da Bearbeitungsvorgänge nicht unterbrochen werden müssen, kann die Bearbeitungszeit und damit die Belastung des Patienten verringert werden.

Durch Anordnung der Sendeeinheit und/oder der Empfangseinheit am Gehäuse oder im Gehäuse kann dem chirurgischen Instrument ferner eine kompakte Bauform verliehen werden. Dies begünstigt die Handhabung des Instrumentes und erlaubt es vorzugsweise, dem Operateur freie Sicht auf das Operationsfeld zu ermöglichen.

Vorzugsweise bilden die Sendeeinheit und die Empfangseinheit eine integrierte Sende- und Empfangseinheit. Dies begünstigt eine kompakte Bauform und konstruktiv einfache Ausgestaltung des Instrumentes.

Von Vorteil ist es, wenn die Sendeeinheit und die Empfangseinheit am Gehäuse oder im Gehäuse angeordnet sind und wenn das mindestens eine Signal von der Sendeeinheit in distaler Richtung auf das zu bearbeitende Körpergewebe aussendbar ist und wenn das reflektierte, in proximaler Richtung verlaufende Signal von der Empfangseinheit erfassbar ist. Von der Sendeeinheit kann mindestens ein Signal in distaler Richtung ausgesandt und von der Empfangseinheit ein reflektiertes Signal, das sich in proximaler Richtung ausbreitet, erfasst werden. Dies kann unter Erzielung einer kompakten Bauform dadurch erreicht werden, dass die Sendeeinheit und die Empfangseinheit am oder im Gehäuse angeordnet sind. Beispielsweise sind Sende- und Empfangseinheit im Gehäuse angeordnet. Es kann auch vorgesehen sein, dass Sende- und Empfangseinheit außerhalb des Gehäuses am Gehäuse angeordnet sind. Denkbar ist auch, dass die Sendeeinheit außenseitig am Gehäuse und die Empfangseinheit im Gehäuse angeordnet ist oder umgekehrt.

In der erfindungsgemäßen Ausgestaltung weist die Vortriebermittlungseinrichtung mindestens ein Reflexionselement auf, von dem das mindestens eine von der Sendeeinheit ausgesandte Signal zumindest teilweise in Richtung der Empfangseinheit reflektierbar ist und das am Werkzeug und/oder am Körpergewebe anordenbar ist. Ein von der Sendeeinheit ausgesandtes Signal kann von dem mindestens einen Reflexionselement in Richtung der Empfangseinheit reflektiert werden, so dass diese das Signal zuverlässiger detektieren kann. Die Anordnung des Reflexionselementes am Werkzeug oder am Körpergewebe erlaubt es auch, die Sende- und die Empfangseinheit am Gehäuse oder im Gehäuse anzuordnen, wie dies vorstehend erwähnt wurde.

Das mindestens eine Reflexionselement kann am Werkzeug und/oder am Körpergewebe festlegbar sein. Beispielsweise kann das Reflexionselement am Körpergewebe durch Verkleben, Verheftung oder Verschraubung festgelegt werden. Dadurch kann das Reflexionselement ortsfest am Körpergewebe verbleiben, wohingegen sich das Instrument unter Vortrieb relativ zum Körpergewebe bewegt.

Alternativ oder ergänzend kann mindestens ein nicht am Werkzeug oder am Körpergewebe anordenbares, festgelegtes oder festlegbares Reflexionselement vorgesehen sein. Dieses Reflexionselement ist beispielsweise ortsfest im Operationssaal angeordnet, und zum Beispiel ist es am Operationstisch festgelegt oder festlegbar. Das mindestens eine Reflexionselement kann insbesondere eine Platte sein oder umfassen.

Das mindestens eine Reflexionselement kann plattenförmig ausgestaltet sein, insbesondere bei Festlegung am Körpergewebe.

Günstigerweise ist das mindestens eine Reflexionselement frei von direkter Verbindung mit dem Gehäuse. Eine Relativbewegung des Gehäuses und des Reflexionselementes lässt sich dadurch auf einfachere Weise erzielen und die Konstruktion des Instrumentes vereinfachen.

Von Vorteil ist es, wenn das mindestens eine Reflexionselement eine Hülse umfasst oder bildet, die vom Werkzeug durchgreifbar ist, wobei das Werkzeug bei Vortrieb des Instrumentes relativ zur Hülse beweglich ist. Ein derartiges Reflexionselement erweist sich insbesondere bei einem Werkzeug in Gestalt eines chirurgischen Bohrers als vorteilhaft. Die Hülse, speziell eine Bohrhülse, kann das Werkzeug umgeben. Bei Bearbeitung des Körpergewebes kann das Werkzeug relativ zur Hülse bewegt werden, zum Beispiel kann ein Bohrer durch die Bohrhülse geführt werden. Die Hülse kann am Körpergewebe festlegbar sein und dadurch während der Bearbeitung in definierter Position am Körpergewebe verbleiben, wohingegen sich das Instrument relativ zum Körpergewebe bewegt.

Von Vorteil ist es, wenn der Vortrieb von der Auswerteeinheit anhand der Laufzeit des mindestens einen Signals von der Sendeeinheit zur Empfangseinheit ermittelbar ist. Die Zeit, die vom Aussenden des Signals bis zum Empfang des Signals vergeht, ist ein Maß für die Distanz der Empfangseinheit von der Sendeeinheit, bezogen auf die Ausbreitungsrichtung des Signals. Diese Distanz, damit auch die Laufzeit des Signals, kann sich bei Vortrieb des Instrumentes ändern, so dass anhand der Laufzeit des Signals der Vortrieb ermittelbar ist.

Alternativ oder ergänzend kann beispielsweise vorgesehen sein, dass der Vortrieb interferometrisch gemessen wird.

Günstig ist es, wenn die Vortriebermittlungseinrichtung eine optische Vortriebermittlungseinrichtung ist und wenn das mindestens eine Signal ein Lichtsignal ist. Bevorzugt wird sichtbares Licht eingesetzt, anhand dessen der Operateur die Funktion der Vortriebermittlungseinrichtung kontrollieren kann. Ist kein Lichtsignal erkennbar, kann der Operateur daraus folgern, dass die Vortriebermittlungseinrichtung deaktiviert ist oder nicht ordnungsgemäß funktioniert.

Als Lichtquelle in der Sendeeinheit kommt insbesondere eine Laserlichtquelle zum Einsatz.

Es kann auch vorgesehen sein, dass die Vortriebermittlungseinrichtung eine sonografische Vortriebermittlungseinrichtung ist und dass das Signal ein Ultraschallsignal ist.

Vorteilhafterweise ist mit der Sendeeinheit eine Mehrzahl von Signalen aussendbar. Bevorzugt ist die Mehrzahl der ausgesandten Signale von der Empfangseinheit erfassbar. Durch Aussenden von mehreren Signalen ist es möglich, die Genauigkeit der Vortriebermittlungseinrichtung zu erhöhen.

Günstig ist es, wenn von der Auswerteeinheit anhand jedes Signals ein individueller Vortrieb des Instrumentes ermittelbar ist und wenn die Auswerteeinheit den Vortrieb als Mittelwert der individuellen Vortriebe ermittelt.

Es kann vorgesehen sein, dass die Signale in Umfangsrichtung des Werkzeuges angeordnet sind und voneinander beabstandet sind. Beispielsweise werden die Signale in distaler Richtung das Werkzeug umgebend ausgesandt. In Umfangsrichtung des Werkzeugs sind die Signale bevorzugt gleichmäßig voneinander beabstandet. Beispielsweise sind eine Mehrzahl von Signalen vorgesehen, die ein Werkzeug in Gestalt eines Bohrers in gleichen Winkelabständen umgeben.

Bei einer bevorzugten Ausführungsform des erfindungsgemäßen Instrumentes ist es von Vorteil, wenn die Sendeeinheit im Gehäuse angeordnet ist und wenn die Vortriebermittlungseinrichtung mindestens einen Signalleiter umfasst, über den das mindestens eine Signal von der Sendeeinheit an einen distalseitig der Sendeeinheit angeordneten Signalaustrittsbereich geleitet wird, an dem das mindestens eine Signal aus dem Gehäuse austritt. Durch den mindestens einen Signalleiter, insbesondere einen Lichtwellenleiter wie beispielsweise eine Glasfaser, kann das mindestens eine Signal von der proximal angeordneten Sendeeinheit zum distalseitig angeordneten Signalaustrittsbereich geleitet werden. Dem Gehäuse kann dadurch distalseitig eine kompakte Bauform verliehen werden, wodurch die Sicht des Operateurs auf das Operationsfeld nicht beeinträchtigt wird.

Der mindestens eine Signalaustrittsbereich ist beispielsweise an einer Werkzeugaufnahme des Instrumentes angeordnet, an der das Werkzeug festlegbar oder festgelegt ist. Dies erweist sich beispielsweise dann als vorteilhaft, wenn eine Mehrzahl von das Werkzeug in Umfangsrichtung umgebenden Signalen, wie vorstehend erwähnt, zum Einsatz kommt.

Vorteilhafterweise umfasst die Vortriebermittlungseinrichtung ein an der Werkzeugaufnahme angeordnetes Signalaustrittselement, und eine Mehrzahl von Signalleitern ist vorzugsweise vorgesehen, die signalwirksam an das Signalaustrittselement angekoppelt sind.

Das Signalaustrittselement kann insbesondere ein Signaleintrittselement bilden, über das die reflektierten Signale eingekoppelt werden können. Die Signale können der Empfangseinheit bevorzugt über die Signalleiter zugeführt werden, welche vorzugsweise eine integrierte Einheit mit der Sendeeinheit bildet.

Günstigerweise ist das Signalaustrittselement ringförmig und umgibt das an der Werkzeugaufnahme angeordnete Werkzeug. Durch das ringförmige Signalaustrittselement können die Signale auch bei drehend angetriebenem Werkzeug (und damit verbunden drehender Werkzeugaufnahme) auf konstruktiv einfache Weise ausgekoppelt werden. Dabei kann vorgesehen sein, dass das Signalaustrittselement drehbeweglich zur Werkzeugaufnahme ist oder drehfest an der Werkzeugaufnahme gehalten ist.

Bei einer weiteren vorteilhaften Ausführungsform des erfindungsgemäßen Instrumentes ist die Sendeeinheit außenseitig am Gehäuse gehalten, vorzugsweise im Bereich eines distalen Endes des Gehäuses. Über die Sendeeinheit kann das mindestens eine Signal konstruktiv einfach ausgesandt werden, ohne dass es hierfür, wie bei der vorstehend erwähnten Ausführungsform, einer Auskopplung des mindestens einen Signals aus dem Gehäuse bedarf.

Auch bei dieser Ausführungsform kann vorgesehen sein, dass die Sendeeinheit eine integrierte Einheit mit der Empfangseinheit bildet, welche dadurch außenseitig am Gehäuse gehalten ist, vorzugsweise im Bereich eines distalen Ende des Gehäuses.

Als vorteilhaft erweist es sich, wenn die Sendeeinheit eine Mehrzahl von Sendeelementen umfasst, die jeweils ein Signal aussenden, wobei den Sendeelementen eine gemeinsame Halterung zum Halten außenseitig am Gehäuse zugeordnet ist. Beispielsweise umgibt die Halterung das Gehäuse ringförmig, insbesondere im Bereich eines distalen Endes des Gehäuses.

Günstig ist es, wenn die Vortriebermittlungseinrichtung eine Einstelleinheit umfasst, mit der die Richtung des mindestens einen Signals und/oder einen Fokus mindestens eines Signals oder einer Mehrzahl von Signalen einstellbar ist. Dadurch kann dem Instrument eine größere Vielseitigkeit verliehen werden. Die vorstehend genannte Halterung kann beispielsweise die Einstelleinheit ausbilden.

Bevorzugt umfasst die Vortriebermittlungseinrichtung eine Eingabeeinheit, über die von einem Benutzer ein Schwellenwert für den Vortrieb eingebbar ist.

Vorteilhafterweise ist die Antriebseinheit bei Erreichen oder Überschreiten eines vorgegebenen oder vorgebbaren Schwellenwertes des Vortriebs abschaltbar. Beispielsweise wird die Antriebseinheit abgeschaltet, wenn der vom Benutzer vorgegebene Schwellenwert oder ein gespeicherter Schwellenwert erreicht oder überschritten ist. Durch Abschalten der Antriebseinheit erweist sich das Instrument als besonders handhabungsfreundlich. Schaltet die Antriebseinheit ab, ist dies ein Hinweis für den Operateur darauf, dass der gewünschte Vortrieb erreicht ist, beispielsweise ein Bohrloch in einem Knochen eine gewünschte Tiefe aufweist.

Von Vorteil ist es, wenn die Vortriebermittlungseinrichtung eine Hinweiseinheit umfasst, über die einem Benutzer den Vortrieb des Instrumentes und/oder das Erreichen oder Überschreiten eines vorgegebenen oder vorgebbaren Schwellenwertes für den Vortrieb signalisierbar ist. Die Hinweiseinheit ist besonders benutzerfreundlich und ermöglicht es, den Operateur auf Erreichen oder Überschreiten eines gewünschten Vortriebs aufmerksam zu machen.

Günstigerweise umfasst oder bildet die Hinweiseinheit zumindest eines der folgenden:
- eine optische Anzeigeeinheit, an der der Vortrieb und/oder das Erreichen oder Überschreiten des Schwellenwertes anzeigbar ist;
- eine Vibrationseinheit zum Bereitstellen einer Vibration des Instrumentes bei Erreichen oder Überschreiten des Schwellenwertes;
- eine akustische Anzeigeeinheit, über die ein dem Vortrieb und/oder dem Erreichen oder Überschreiten des Schwellenwertes zugeordnetes Geräusch ausgebbar ist.

Vorstehende Ausgestaltung ermöglicht es, dem Benutzer den Vortrieb und/oder das Erreichen oder Überschreiten eines Schwellenwertes optisch, haptisch und/oder akustisch zu signalisieren.

Die Hinweiseinheit ist bevorzugt im Gehäuse angeordnet oder, beispielsweise im Falle einer optischen oder akustischen Anzeigeeinheit, an einer Gehäusewand angebracht oder in diese integriert.

Das Instrument kann eine Einrichtung zum Ermitteln eines Drehmomentes und/oder einer Drehmomentänderung der Antriebseinheit aufweisen. Bei einer Änderung des Drehmomentes kann die Antriebseinheit deaktiviert werden. Diese Drehmomentänderung ist zum Beispiel darauf zurückzuführen, dass ein Knochen durchbohrt worden ist und sich dementsprechend der Widerstand ändert, den das Körpergewebe dem Werkzeug entgegensetzt.

Es kann vorgesehen sein, dass an der Hinweiseinheit einem Operateur der Vortrieb oder dessen Änderung signalisiert wird (beispielsweise an einer Anzeigeeinheit angezeigt), wenn eine Drehmomentänderung auftritt, insbesondere wenn die Antriebseinheit infolge der Änderung des Drehmomentes deaktiviert wird. Beispielsweise kann dabei die Dicke eines durchbohrten Knochens signalisiert werden.

Wie eingangs erwähnt, kann vorgesehen sein, dass das Instrument eine chirurgische Bohrvorrichtung ist, wobei das chirurgische Werkzeug ein chirurgischer Bohrer ist.

Es ist denkbar, dass das Instrument eine chirurgische Bohrvorrichtung ist, wobei das chirurgische Werkzeug ein Kirschnerdraht ist.

Denkbar ist auch, dass das Instrument eine chirurgische Fräsvorrichtung ist, wobei das chirurgische Werkzeug ein chirurgischer Fräskopf ist.

Weiter ist denkbar, dass das Instrument eine chirurgische Schraubvorrichtung ist, wobei das chirurgische Werkzeug ein Schraubwerkzeug zum Zusammenwirken mit einer chirurgischen Schraube ist.

Die nachfolgende Beschreibung bevorzugter Ausführungsformen der Erfindung dient im Zusammenhang mit der Zeichnung der näheren Erläuterung der Erfindung. Es zeigen:
- Fig. 1: eine schematische Darstellung einer ersten bevorzugten Ausführungsform eines erfindungsgemäßen Instrumentes;
- Fig. 2: eine Schnittansicht längs der Linie 2-2 in Figur 1 und
- Fig. 3: eine schematische Darstellung einer zweiten bevorzugten Ausführungsform eines erfindungsgemäßen Instrumentes.

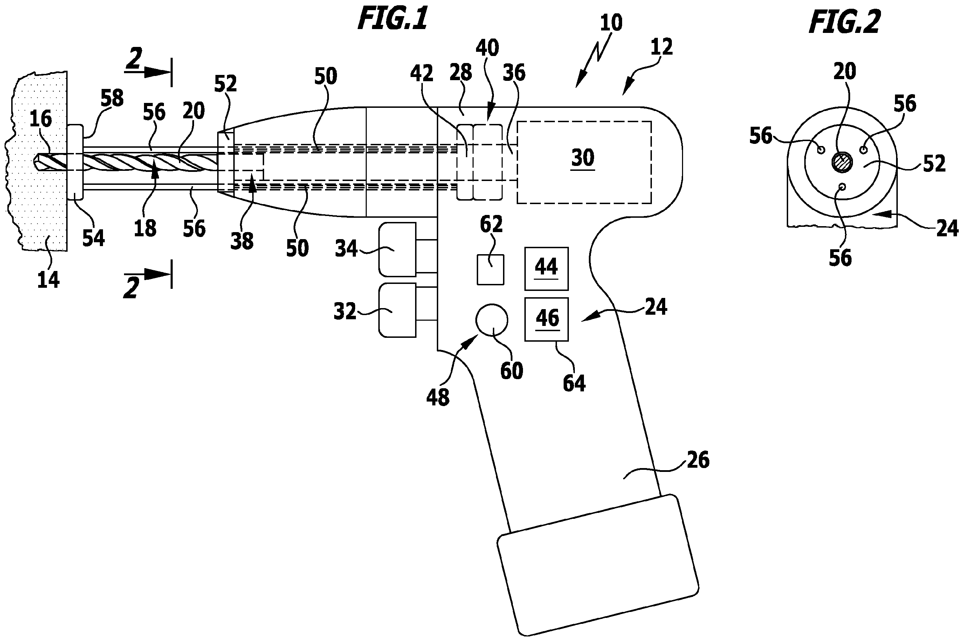

Figur 1 zeigt in schematischer Darstellung eine insgesamt mit dem Bezugszeichen 10 belegte vorteilhafte Ausführungsform eines erfindungsgemäßen chirurgischen Instrumentes. Das Instrument 10 ist insbesondere eine chirurgische Bohrvorrichtung 12. Mit der Bohrvorrichtung 12 kann ein Körpergewebe 14 bearbeitet werden. Bei dem Körpergewebe 14 handelt es sich insbesondere um einen Knochen, in den mit der Bohrvorrichtung 12 ein Bohrloch 16 zur Aufnahme eines Implantates (nicht gezeigt) gebohrt werden kann. Zu diesem Zweck umfasst die Bohrvorrichtung 12 ein chirurgisches Werkzeug 18, vorliegend in Gestalt eines chirurgischen Bohrers 20.

Die Bohrvorrichtung 12 umfasst ein insgesamt mit dem Bezugszeichen 24 belegtes Gehäuse. Das Gehäuse 24 umfasst zwei im Winkel zueinander ausgerichtete Gehäuseabschnitte 26 und 28. Der Gehäuseabschnitt 26 bildet ein Griffelement für einen Operateur zum Greifen der Bohrvorrichtung 12. Der Gehäuseabschnitt 28 ist in proximal-distaler Richtung ausgerichtet.

"Proximal" und "distal" sind vorliegend so aufzufassen, dass ein Benutzer die Bohrvorrichtung 12 von proximal ergreift und mit der distalen Seite der Bohrvorrichtung 12 auf das Körpergewebe 14 einwirkt.

Im Gehäuseabschnitt 28 ist proximal eine Antriebseinheit 30 angeordnet. Der Operateur kann mit am Gehäuseabschnitt 26 angeordneten Bedienelementen 32, 34, die in Wirkverbindung mit der Antriebseinheit 30 stehen, die Antriebseinheit 30 aktivieren oder deaktivieren. Die Antriebseinheit 30 ist über eine Welle 36 mit einer Werkzeugaufnahme 38 gekoppelt. Die Werkzeugaufnahme 38 ist distalseitig am Gehäuseabschnitt 28 angeordnet. Der Bohrer 20 ist lösbar in der Werkzeugaufnahme 38 gehalten und in proximal-distaler Richtung ausgerichtet.

Bei aktivierter Antriebseinheit 30 kann der Bohrer 20 drehend angetrieben werden, um den Knochen 14 zu bearbeiten. Dabei kann ein Vortrieb der Bohrvorrichtung 12 dadurch erzielt werden, dass der Bohrer 20 beim Bohren des Bohrloches 16 in den Knochen 14 eindringt. Um den Vortrieb der Bohrvorrichtung 12 relativ zum Knochen 14 handhabungsfreundlich und benutzerfreundlich zu ermitteln, umfasst die Bohrvorrichtung 12 eine Vortriebermittlungseinrichtung 40. Die Vortriebermittlungseinrichtung 40 weist eine integrierte Sende- und Empfangseinheit 42, eine Auswerteeinheit 44, eine Hinweiseinheit 46, eine Eingabeeinheit 48, Signalleiter 50, ein Signalaustrittselement 52 und ein Reflexionselement 54 auf.

Die Sende- und Empfangseinheit 42 ist im Gehäuse 26 proximal der Antriebseinheit 30 angeordnet und stellt Signale bereit, vorliegend bevorzugt in Form von Lichtsignalen. Zu diesem Zweck kann die Sende- und Empfangseinheit eine oder mehrere Laserlichtquellen umfassen, die insbesondere sichtbares Licht bereitstellen. Entsprechend ihrer Bezeichnung kann die Sende- und Empfangseinheit sowohl Signale bereitstellen als auch empfangen. Beim Aussenden und Empfangen eines Signals kann der Auswerteeinheit 44, die mit der Sende- und Empfangseinheit 42 in Wirkverbindung steht, ein diesbezügliches Steueroder Hinweissignal übermittelt werden.

An die Sende-und Empfangseinheit 42 signalwirksam angekoppelt sind (vorliegend drei) Signalleiter 50. Die Signalleiter 50 sind ausgestaltet als Glasfasern. Durch jeden Signalleiter 50 kann ein von der Sende- und Empfangseinheit bereitgestelltes Signal in distaler Richtung durch den Gehäuseabschnitt 26 geleitet werden. Die Signalleiter 50 sind signalwirksam an das Signalaustrittselement 52 angekoppelt. Das Signalaustrittselement 52 ist distal am Gehäuseabschnitt 28 angeordnet, und zwar an der Werkzeugaufnahme 38.

Das Signalaustrittselement 52 ist vorliegend ringförmig und umgibt den Bohrer 20. Es kann vorgesehen sein, dass das Signalaustrittselement 52 drehfest an der Werkzeugaufnahme 38 gehalten ist oder drehbeweglich relativ zu dieser ist. In beiden Fällen kann durch die signalwirksame Ankopplung der Signalleiter 50 das jeweilige Signal über das Signalaustrittselement 52 aus dem Gehäuse 24 austreten und in distaler Richtung auf den Knochen 14 ausgesandt werden. Dies wird dadurch ermöglicht, dass das Signalaustrittselement 52 transparent für die verwendeten Signale ist. Beispielsweise handelt es sich bei Verwendung von Lichtsignalen um ein Signalaustrittselement 52 aus Glas oder einem optisch transparenten Kunststoffmaterial.

Vorliegend sind die Signalleiter 50 so angeordnet und an das Signalaustrittselement 52 angekoppelt, dass die aus dem Gehäuse 24 austretenden Lichtsignale den Bohrer 20 in dessen Umfangsrichtung umgeben. Dies ist in Figur 2 gezeigt, in der die Signale mit dem Bezugszeichen 56 gekennzeichnet sind. In Umfangsrichtung des Bohrers 20 weisen die Signale 56 identische Winkelabstände voneinander auf.

Das Reflexionselement 54 ist im vorliegenden Fall ein optisch wirksames Reflexionselement, das beispielsweise eine verspiegelte, dem Signalaustrittselement 52 zugewandte Stirnseite 58 aufweisen kann. Das Reflexionselement 54 ist ausgestaltet als Hülse und insbesondere als Bohrhülse, von der der Bohrer 20 geführt ist. Ferner ist das Reflexionselement 54 am Knochen 14 festlegbar, beispielsweise durch Verklebung oder Verheftung.

Anstelle einer Bohrhülse könnte auch ein insbesondere plattenförmiges Reflexionselement vorgesehen sein, das bevorzugt am Knochen 14 festlegbar ist.

Denkbar ist auch, dass ein Reflexionselement nicht unmittelbar am zu bearbeiteten Knochen 14 anliegt. Beispielsweist kann vorgesehen sein, dass ein Reflexionselement an Weichkörpergewebe anliegt, welches oberhalb des Knochens angeordnet ist und in dem ein Zugang vorgesehen ist, durch den hindurch mit dem Bohrer 20 auf den Knochen 14 eingewirkt werden kann. Dies ist in der Zeichnung nicht dargestellt.

Signale 56, die auf das Reflexionselement 54 fallen, können von diesem wieder in Richtung auf das Signalaustrittselement 52 reflektiert werden. Die von der Sende- und Empfangseinheit 42 ausgesandten Signale, die sich zunächst in distaler Richtung ausbreiten, werden dadurch in proximaler Richtung zurückgeworfen. Durch das transparente Signalaustrittselement 52 können die Signale wieder in die Signalleiter 52 eingekoppelt und von der Sende- und Empfangseinheit 42 erfasst werden. Das Signalaustrittselement 52 ist daher auch ein Signaleintrittselement.

Das Reflexionselement 54 ist relativ zum Bohrer 20 beweglich. Bei der Bearbeitung des Knochens 14, wenn die Bohrvorrichtung 12 vorgetrieben und relativ zu diesem bewegt wird, ändert sich dadurch der Abstand des Reflexionselementes 54 vom Signalaustrittselement 52. Die Distanz, die von den Signalen 56 durchlaufen wird, nachdem sie von der Sende- und Empfangseinheit 42 ausgesandt und bevor sie von derselben wieder empfangen werden, ändert sich dadurch. Diese Distanz entspricht dem Signalweg, insbesondere dem optischen Lichtweg bei Verwendung einer optischen Sende- und Empfangseinheit von Lichtsignalen.

Diese Distanz kann beispielsweise anhand der Laufzeit der Signale 56 ermittelt werden. Zu diesem Zweck kann vorgesehen sein, dass die Signale 56 getaktet, insbesondere periodisch, und/oder gepulst abgegeben und auch wieder empfangen werden können.

Die Auswerteeinheit 44 ermöglicht es, anhand der Signale 56 anhand der Änderung der Distanz bei der Bearbeitung des Knochens 14 den Vortrieb der Bohrvorrichtung 12 zu ermitteln. Dies gibt die Möglichkeit, in deren Betrieb die Tiefe des Bohrloches 16 zu ermitteln. Dies erweist sich als besonders handhabungsund benutzerfreundlich. Dem Operateur bleibt es erspart, die Bearbeitung des Knochens 14 zu unterbrechen, um die Tiefe des Bohrloches 16 zu kontrollieren. Stattdessen kann dies während der Bearbeitung des Knochens 14 von der Auswerteeinheit 44 ermittelt werden.

Die Auswerteeinheit 44 kann anhand jedes Signales 56 individuell einen Vortrieb ermitteln und den Vortrieb des Instrumentes als Mittelwert der individuell ermittelten Vortriebe bestimmen, um die Genauigkeit zu steigern. Die Auswerteeinheit 44 ist mit der Eingabeeinheit 48 und mit der Hinweiseinheit 46 in Wirkverbindung. Über die Eingabeeinheit 48 kann der Operateur der Auswerteeinheit 44 einen Schwellenwert für den Vortrieb vorgeben. Es kann vorgesehen sein, dass die Antriebseinheit 30 unter Ansteuerung durch die Auswerteeinheit 44 bei Erreichen oder Überschreiten des vorgegebenen Schwellenwertes oder eines gespeicherten Schwellenwertes abgeschaltet wird. Zum Vorgeben des Schwellenwertes kann die Eingabeeinheit 48 ein Einstellelement 60 aufweisen. Weiter kann die Eingabeeinheit 48 ein Rückstellelement 62 aufweisen. Über das Rückstellelement 62 kann der Auswerteeinheit 44 ein Nullpunkt des Vortriebs vorgegeben werden, beispielsweise bevor die Bearbeitung des Knochens 14 begonnen wird und der Bohrer 20 an diesem anliegt.

Die Hinweiseinheit 46 ist insbesondere eine optische Hinweiseinheit mit einer optischen Anzeigeeinheit 64. Die Anzeigeeinheit 64 ist beispielsweise an eine Wand des Gehäuses 24 integriert und sie kann insbesondere ein LCD-Display umfassen. An der Anzeigeeinheit 64 kann dem Benutzer der momentane Vortrieb und gegebenenfalls ein vorgegebener Schwellenwert für den Vortrieb angezeigt werden.

Unter Einsatz der Hinweiseinheit 46 kann dem Operateur dadurch signalisiert werden, wie der aktuelle Vortrieb ist und/oder ob ein vorgegebener Schwellenwert des Vortriebs erreicht oder überschritten wurde. Dies erlaubt es dem Operateur auf benutzerfreundliche Weise festzustellen, wie groß der Vortrieb des Instrumentes 10 und damit die Tiefe des Bohrloches 16 ist.

Es kann vorgesehen sein, dass die Hinweiseinheit 46 eine akustische Anzeigeeinheit ist, über die ein dem Vortrieb und/oder dem Erreichen oder Überschreiten des Schwellenwertes zugeordnetes Geräusch ausgegeben werden kann.

Weiter ist denkbar, dass die Hinweiseinheit 46 eine haptische Hinweiseinheit ist. Beispielsweise umfasst eine solche eine Vibrationseinheit, die eine Vibration des Gehäuses 24 hervorrufen kann, wenn ein vorgegebener Vortrieb erreicht oder überschritten worden ist.

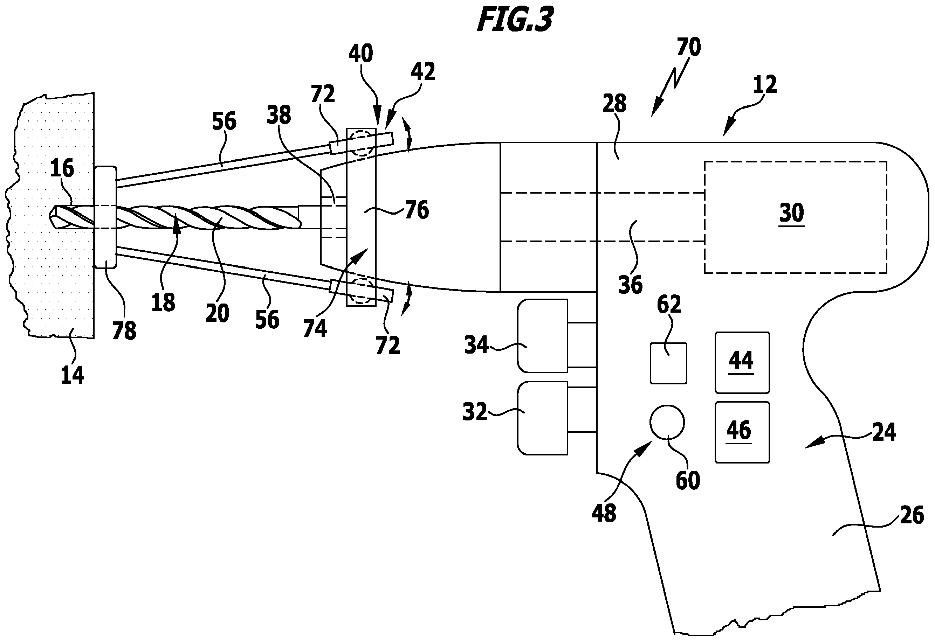

Bei einer in Figur 3 dargestellten und insgesamt mit dem Bezugszeichen 70 belegten vorteilhaften Ausführungsform eines chirurgischen Instrumentes sind Merkmale oder Bauteile, die gleich oder gleichwirkend zu Merkmalen oder Bauteilen des Instrumentes 10 sind, mit denselben Bezugszeichen belegt. Die mit dem Instrument 10 erzielbaren Vorteile können mit dem Instrument 70 ebenfalls erzielt werden, so dass diesbezüglich auf die voranstehenden Erläuterungen verwiesen werden kann.

Beim Instrument 70 umfasst die Sende- und Empfangseinheit 42 eine Mehrzahl von Sendeelementen 72, die jeweils ein Signal 56 aussenden. Insgesamt sind drei Sendeelemente 72 vorgesehen, die außenseitig am Gehäuse 24 angeordnet sind. Insbesondere sind die Sendeelemente 72 im Bereich eines distalen Endes des Gehäuseabschnittes 26 angeordnet, wobei sie die Werkzeugaufnahme 38 außenseitig umgeben. In Umfangsrichtung des Gehäuseabschnittes 26 weisen die Sendeelemente 72 identische Winkelabstände auf (nur zwei Sendeelemente 72 sind gezeigt).

Die Sendeelemente 72 sind zugleich Empfangselemente. Auch das Instrument 70 weist damit eine integrierte Sende- und Empfangseinheit auf.

Den Sendeelementen 72 ist eine gemeinsame Halterung 74 zugeordnet. Die Halterung 74 umfasst vorliegend insbesondere einen Ring 76. Der Ring 76 bildet eine Einstelleinheit für die Sende- und Empfangseinheit 42 und ist so relativ zum Gehäuseabschnitt 26 drehbar, dass die Neigung der Sendeelemente 72 relativ zum Gehäuseabschnitt 26 verändert werden kann. Dementsprechend kann die Richtung, unter denen die Signale 56 ausgesandt werden, verändert werden. Dies gibt die Möglichkeit, die Signale 56 auf das Reflexionselement 54 auszurichten um sicherzustellen, dass dieses von den Signalen 56 auch getroffen werden kann. Anstelle des Reflexionselementes 54 kann beim Instrument 70 insbesondere ein Streuelement 78 vorgesehen sein, so dass einfallende Signale 56 so gestreut werden können, dass sie von einem Sendeelement, 72 zum Beispiel dem aussendenden, wieder erfasst werden können.

Bei den Instrumenten 10 und 70 ist es selbstverständlich möglich, dass anstelle der integrierten Sende- und Empfangseinheit Sendeeinheit und Empfangseinheit voneinander getrennt sein können.

## Patentansprüche

1. Chirurgisches Instrument, insbesondere Bohrvorrichtung, Fräsvorrichtung oder Schraubvorrichtung, umfassend ein Gehäuse (24) und eine darin angeordnete Antriebseinheit (30), wobei distal am Gehäuse (24) ein chirurgisches Werkzeug (18) zum Einwirken auf ein zu bearbeitendes Körpergewebe (14), insbesondere Knochen, festlegbar oder festgelegt ist, welches Werkzeug (18) von der Antriebseinheit (30) drehend oder oszillierend antreibbar ist und wobei bei angetriebenem Werkzeug (18) ein Vortrieb des Instrumentes (10; 70) relativ zum Körpergewebe (14) erzielbar ist, wobei das Instrument (10; 70) eine Vortriebermittlungseinrichtung (40) umfasst, mit der der Vortrieb des Instruments (10; 70) relativ zum Körpergewebe (14) im Betrieb des Instrumentes (10; 70) ermittelbar ist und die eine Sendeeinheit (42) zum Aussenden mindestens eines Signals (56), eine Empfangseinheit (42) zum Erfassen des mindestens einen Signals (56) und eine Auswerteinheit (44) zum Ermitteln des Vortriebs anhand des mindestens einen Signals (56) umfasst, und wobei zumindest die Sendeeinheit (42) oder die Empfangseinheit (42) an dem Gehäuse (24) oder im Gehäuse (24) angeordnet ist, **dadurch gekennzeichnet, dass** die Vortriebermittlungseinrichtung (40) mindestens ein Reflexionselement (54) aufweist, von dem das mindestens eine von der Sendeeinheit (42) ausgesandte Signal (56) zumindest teilweise in Richtung der Empfangseinheit (42) reflektierbar ist und das am Werkzeug (18) und/oder am Körpergewebe (14) anordenbar ist.

2. Instrument nach Anspruch 1,
**dadurch gekennzeichnet, dass**
die Sendeeinheit (42) und die Empfangseinheit (42) eine integrierte Sende- und Empfangseinheit (42) bilden.

3. Instrument nach Anspruch 1 oder 2,
**dadurch gekennzeichnet, dass**
die Sendeeinheit (42) und die Empfangseinheit (42) am Gehäuse (24) oder im Gehäuse (24) angeordnet sind und dass das mindestens eine Signal (56) von der Sendeeinheit (42) in distaler Richtung auf das zu bearbeitende Körpergewebe (14) ausendbar ist und dass das reflektierte, in proximaler Richtung verlaufende Signal (56) von der Empfangseinheit (42) erfassbar ist.

4. Instrument nach einem der vorhergehenden Ansprüche,
**dadurch gekennzeichnet, dass**
das mindestens eine Reflexionselement (54) am Werkzeug (18) und/oder am Körpergewebe (14) festlegbar ist.

5. Instrument nach einem der vorhergehenden Ansprüche,
**dadurch gekennzeichnet, dass**
das mindestens eine Reflexionselement (54) eine Hülse umfasst oder bildet, die vom Werkzeug (18) durchgreifbar ist, wobei das Werkzeug (18) bei Vortrieb des Instruments (10; 70) relativ zur Hülse beweglich ist.

6. Instrument nach einem der voranstehenden Ansprüche,
**dadurch gekennzeichnet, dass**
die Vortriebermittlungseinrichtung (40) eine optische Vortriebermittlungseinrichtung ist (40) und dass das mindestens eine Signal (56) ein Lichtsignal ist und/oderdass die Vortriebermittlungseinrichtung eine sonografische Vortriebermittlungseinrichtung ist und dass das mindestens eine Signal ein Ultraschallsignal ist.

7. Instrument nach einem der voranstehenden Ansprüche,
**dadurch gekennzeichnet, dass**
mit der Sendeeinheit (42) eine Mehrzahl von Signalen (56) aussendbar und bevorzugt von der Empfangseinheit (42) erfassbar ist.

8. Instrument nach einem der voranstehenden Ansprüche,
**dadurch gekennzeichnet, dass**
die Sendeeinheit (42) im Gehäuse (24) angeordnet ist und dass die Vortriebermittlungseinrichtung (40) mindestens einen Signalleiter (50) umfasst, über den das mindestens eine Signal (56) von der Sendeeinheit (42) an einen distalseitig der Sendeeinheit (42) angeordneten Signalaustrittsbereich geleitet wird, an dem das mindestens eine Signal (56) aus dem Gehäuse (24) austritt.

9. Instrument nach Anspruch 8,
**dadurch gekennzeichnet, dass**
der mindestens eine Signalaustrittsbereich an einer Werkzeugaufnahme (38) des Instrumentes (10) angeordnet ist, an der das Werkzeug (18) festlegbar oder festgelegt ist.

10. Instrument nach einem der voranstehenden Ansprüche,
**dadurch gekennzeichnet, dass**
die Sendeeinheit (42) außenseitig am Gehäuse gehalten ist, vorzugsweise im Bereich eines distalen Endes des Gehäuses (24).

11. Instrument nach einem der voranstehenden Ansprüche,
**dadurch gekennzeichnet, dass**
die Vortriebermittlungseinrichtung (40) eine Einstelleinheit umfasst, mit der die Richtung des mindestens einen Signals (56) und/oder ein Fokus mindestens eines Signals (56) oder einer Mehrzahl von Signalen (56) einstellbar ist.

12. Instrument nach einem der voranstehenden Ansprüche,
**dadurch gekennzeichnet, dass**
die Vortriebermittlungseinrichtung (40) eine Hinweiseinheit (46) umfasst, über die einem Benutzer der Vortrieb des Instruments (10; 70) und/oder das Erreichen oder Überschreiten eines vorgegebenen oder vorgebbaren Schwellenwertes für den Vortrieb signalisierbar ist.

13. Instrument nach einem der voranstehenden Ansprüche,
**gekennzeichnet durch**
eine Einrichtung zum Ermitteln eines Drehmomentes und/oder einer Drehmomentänderung der Antriebseinheit.

14. Instrument nach den Ansprüchen 12 und 13, **dadurch gekennzeichnet, dass**
vorgesehen ist, dass an der Hinweiseinheit einem Operateur der Vortrieb oder dessen Änderung signalisiert wird, wenn eine Drehmomentänderung auftritt.

## Claims

1. Surgical instrument, in particular a drilling device, milling device or screwing device, comprising a housing (24) and a drive unit (30) arranged therein, wherein a surgical tool (18) for acting on a body tissue (14) to be worked on, in particular bone, is fixable or fixed distally on the housing (24), which tool (18) is drivable in rotation or oscillation by the drive unit (30), and wherein an advance of the instrument (10; 70) relative to the body tissue (14) is achievable when the tool (18) is driven, wherein the instrument (10; 70) comprises an advance-determining device (40) with which the advance of the instrument (10; 70) relative to the body tissue (14) is determinable during the operation of the instrument (10; 70), and which comprises a transmitting unit (42) for transmitting at least one signal (56), a receiving unit (42) for detecting the at least one signal (56), and an evaluation unit (44) for determining the advance on the basis of the at least one signal (56), and wherein at least the transmitting unit (42) or the receiving unit (42) is arranged on the housing (24) or in the housing (24), **characterized in that** the advance-determining device (40) has at least one reflection element (54) from which the at least one signal (56) transmitted by the transmitting unit (42) can be reflected at least partially in the direction of the receiving unit (42), and which can be arranged on the tool (18) and/or on the body tissue (14).

2. Instrument according to Claim 1, **characterized in that** the transmitting unit (42) and the receiving unit (42) form an integrated transmitting and receiving unit (42).

3. Instrument according to Claim 1 or 2, **characterized in that** the transmitting unit (42) and the receiving unit (42) are arranged on the housing (24) or in the housing (24), and **in that** the at least one signal (56) is transmittable by the transmitting unit (42) in the distal direction to the body tissue (14) that is to be worked on, and **in that** the reflected signal (56) running in the proximal direction is detectable by the receiving unit (42).

4. Instrument according to one of the preceding claims, **characterized in that** the at least one reflection element (54) is fixable on the tool (18) and/or on the body tissue (14).

5. Instrument according to one of the preceding claims, **characterized in that** the at least one reflection element (54) comprises or forms a sleeve through which the tool (18) can pass, wherein the tool (18) is movable relative to the sleeve during advance of the instrument (10; 70).

6. Instrument according to one of the preceding claims, **characterized in that** the advance-determining device (40) is an optical advance-determining device (40), and **in that** the at least one signal (56) is a light signal, and/or **in that** the advance-determining device is a sonographic advance-determining device, and **in that** the at least one signal is an ultrasonic signal.

7. Instrument according to one of the preceding claims, **characterized in that** a plurality of signals (56) are transmittable by the transmitting unit (42) and preferably detectable by the receiving unit (42).

8. Instrument according to one of the preceding claims, **characterized in that** the transmitting unit (42) is arranged in the housing (24), and **in that** the advance-determining device (40) comprises at least one signal conductor (50) via which the at least one signal (56) is conducted from the transmitting unit (42) to a signal output region arranged distally from the transmitting unit (42) and at which the at least one signal (56) exits the housing (24).

9. Instrument according to Claim 8, **characterized in that** the at least one signal output region is arranged at a tool holder (38) of the instrument (10), to which tool holder (38) the tool (18) is fixable or fixed.

10. Instrument according to one of the preceding claims, **characterized in that** the transmitting unit (42) is held on the outside of the housing, preferably in the region of a distal end of the housing (24).

11. Instrument according to one of the preceding claims, **characterized in that** the advance-determining device (40) comprises an adjusting unit with which the direction of the at least one signal (56) and/or a focus of at least one signal (56) or of a plurality of signals (56) is adjustable.

12. Instrument according to one of the preceding claims, **characterized in that** the advance-determining device (40) comprises an indicator unit (46) by which a user can be notified of the advance of the instrument (10; 70) and/or of the attaining or exceeding of a predefined or predefinable threshold value for the advance.

13. Instrument according to one of the preceding claims, **characterized by** a device for determining a torque and/or a change of torque of the drive unit.

14. Instrument according to Claims 12 and 13, **characterized in that** provision is made that the advance, or the change thereof, is indicated to an operator on the indicator unit when a change of torque occurs.

## Revendications

1. Instrument chirurgical, notamment dispositif de forage, dispositif de fraisage ou dispositif de vissage, comprenant un boîtier (24) et une unité d'entraînement (30) disposée dans celui-ci, un outil chirurgical (18) destiné à agir sur un tissu corporel à traiter (14), en particulier des os, étant fixé ou pouvant être fixé au boîtier (24) du côté distal, lequel outil (18) pouvant être entraîné en rotation ou en oscillation par l'unité d'entraînement (30) et l'avancement de l'instrument (10 ; 70) par rapport au tissu corporel (14) pouvant être obtenu pendant l'entraînement de l'outil (18), l'instrument (10 ; 70) comprenant un moyen de détermination d'avancement (40) avec lequel l'avancement de l'instrument (10 ; 70) par rapport au tissu corporel (14) peut être déterminé pendant le fonctionnement de l'instrument (10 ; 70) et qui comprend une unité d'émission (42) destinée à émettre au moins un signal (56), une unité de réception (42) destinée à détecter l'au moins un signal (56) et une unité d'évaluation (44) destinée à déterminer l'avancement sur la base de l'au moins un signal (56) et au moins l'unité d'émission (42) ou l'unité de réception (42) étant disposée sur le boîtier (24) ou dans le boîtier (24), **caractérisé en ce que** le moyen de détermination d'avancement (40) comprend au moins un élément de réflexion (54) permettant de réfléchir le signal (56), émis par l'unité d'émission (42), au moins en partie en direction de l'unité de réception (42) et pouvant être disposé au niveau de l'outil (18) et/ou du tissu corporel (14).

2. Instrument selon la revendication 1, **caractérisé en ce que** l'unité d'émission (42) et l'unité de réception (42) forment une unité d'émission et de réception intégrée (42).

3. Instrument selon la revendication 1 ou 2, **caractérisé en ce que** l'unité d'émission (42) et l'unité de réception (42) sont disposées sur le boîtier (24) ou dans le boîtier (24) et **en ce que** l'au moins un signal (56) peut être émis par l'unité d'émission (42) dans la direction distale vers le tissu corporel à traiter (14) et **en ce que** le signal réfléchi (56), s'étendant dans la direction proximale, peut être détecté par l'unité de réception (42).

4. Instrument selon l'une des revendications précédentes, **caractérisé en ce que** l'au moins un élément de réflexion (54) peut être fixé sur l'outil (18) et/ou sur le tissu corporel (14).

5. Instrument selon l'une des revendications précédentes, **caractérisé en ce que** l'au moins un élément de réflexion (54) comprend ou forme un manchon pouvant être traversé par l'outil (18), l'outil (18) étant mobile par rapport au manchon pendant l'avancement de l'instrument (10 ; 70).

6. Instrument selon l'une des revendications précédentes, **caractérisé en ce que** le moyen de détermination d'avancement (40) est un moyen de détermination d'avancement optique (40) et **en ce que** l'au moins un signal (56) est un signal lumineux et/ou **en ce que** le moyen de détermination d'avancement est un moyen de détermination d'avancement sonographique et **en ce que** l'au moins un signal est un signal ultrasonore.

7. Instrument selon l'une des revendications précédentes, **caractérisé en ce qu'**une pluralité de signaux (56) peut être émise avec l'unité d'émission (42) et peut être détectée de préférence par l'unité de réception (42).

8. Instrument selon l'une des revendications précédentes, **caractérisé en ce que** l'unité d'émission (42) est disposée dans le boîtier (24) et **en ce que** le moyen de détermination d'avancement (40) comprend au moins un conducteur de signal (50) permettant de conduire l'au moins un signal (56) de l'unité d'émission (42) vers une région de sortie de signal qui est disposée du côté distal de l'unité d'émission (42) et au niveau de laquelle l'au moins un signal (56) sort du boîtier (24).

9. Instrument selon la revendication 8, **caractérisé en ce que** l'au moins une région de sortie de signal est disposée au niveau d'un porte-outil (38) de l'instrument (10) auquel l'outil (18) est fixé ou peut être fixé.

10. Instrument selon l'une des revendications précédentes, **caractérisé en ce que** l'unité d'émission (42) est maintenue sur le boîtier du côté extérieur, de préférence au niveau d'une extrémité distale du boîtier (24).

11. Instrument selon l'une des revendications précédentes, **caractérisé en ce que** le moyen de détermination d'avancement (40) comprend une unité de réglage permettant de régler la direction de l'au moins un signal (56) et/ou la focalisation d'au moins un signal (56) ou d'une pluralité de signaux (56).

12. Instrument selon l'une des revendications précédentes, **caractérisé en ce que** le moyen de détermination d'avancement (40) comprend une unité de référence (46) permettant de signaler à un utilisateur que l'instrument (10 ; 70) avance et/ou qu'un seuil d'avancement prédéterminé ou prédéterminable est atteint ou dépassé.

13. Instrument selon l'une des revendications précédentes, **caractérisé par** un moyen de détermination d'un couple et/ou d'une variation de couple de l'unité d'entraînement.

14. Instrument selon les revendications 12 et 13, **caractérisé en ce qu'**il est prévu de signaler l'avancement ou sa variation à un opérateur sur l'unité de référence lorsqu'une variation de couple se produit.
